# EUROPEAN PATENT APPLICATION

(11) **EP 1 122 305 A1**
(43) Date of publication of application: **08.08.2001**
(21) Application number: 00200309.3
(22) Date of filing: 31.01.2000
(51) Int. Cl.: C12N 9/06, C12P 17/18, C12N 1/20, C07D 503/00

(54) **Glutamate dehydrogenase-transformed streptomyces clavuligerus with an improved capacity to assimilate ammonia**

(71) Applicant: DSM N.V., 6411 TE Heerlen (NL)
(72) Inventor: Luiten, Rudolf,Gijsbertus,Marie, 2317 KR Leiden (NL); De Laat, Wilhelmus, Theodorus, Antonius,Maria, 4817 KW Breda (NL); Koekman, Bertus,Pieter, 2636 BG Schipluiden (NL); Streekstra, Hugo, 1017 SP Amsterdam (NL); Van Wezel, Gilles, Philippus, 2318 MA Leiden (NL); Zuurmond, Anne-Marie, 2564 LE Den Haag (NL)
(74) Representative: Visser-Luirink, Gesina, Dr.

(57) **Abstract**

The present invention relates to mutant micro-organisms with an improved capacity to assimilate ammonia and to an improved fermentation process for the production of secondary metabolites, more specifically, clavulanic acid.

SEQUENCE LISTING

<110> DSM N.V.

<120> MUTANT MICRO-ORGANISMS WITH AN IMPROVED CAPACITY TO
      ASSIMILATE AMMONIA

<130> GDH

<140> EP-2972P
<141> 2000-01-31

<160> 2

<170> PatentIn Ver. 2.1

<210> 1
<211> 30
<212> DNA
<213> Corynebacterium glutamicum

<400> 1
gtcgaattca tatgacagtt gatgagcagg                                   30


<210> 2
<211> 28
<212> DNA
<213> Corynebacterium glutamicum

<400> 2
gtcaagcttg tggatcggat aattgacc                                     28

## Description

### Field of the invention

The present invention relates to mutant micro-organisms with an improved capacity to assimilate ammonia and to an improved fermentation process for the production of secondary metabolites, more specifically, clavulanic acid.

### Background of the invention

Many antibiotic compounds are manufactured by culturing the antibiotic producing micro-organism under controlled conditions in large, industrial-scale fermentors of 10-300 m³. The micro-organism is grown in a medium comprising, among others, a usually complex source of nitrogen, such as a protein hydrolysate. Although these nitrogen sources provide the production organism with readily consumable nutrients, it will be obvious, that said nutrients are more expensive than simple inorganic sources of nitrogen like ammonia, and that they may constitute a substantial fraction of the variable costs of the fermentation.

The use of simple nitrogen sources, however, may adversely affect product yield in secondary metabolite fermentations. This will especially be the case when the micro-organism has a poor capacity to assimilate molecular ammonia, resulting in poor growth. In order to solve this problem, said nitrogen assimilation capacity has to be improved.

In micro-organisms there are two pathways for the assimilation of ammonia (NH₃) or ammonium (NH₄⁺). One system involves glutamate dehydrogenase (GDH, EC 1.4.1.2), which catalyses the reductive amination of 2-ketoglutarate to glutamate. The second system involves glutamine synthetase (GS, EC 6.3.1.2), which catalyses the amination of glutamate to glutamine, and glutamate synthase (GOGAT, EC 1.4.1.14), which transfers the side chain NH₂-group of glutamine to 2-ketoglutarate, to form two molecules of glutamate. Many micro-organisms, such as *Escherichia coli, Klebsiella pneumoniae*, *Corynebacterium glutamicum*, *Saccharomyces cerevisiae* and *Aspergillus nidulans,* possess both pathways. In these cases, the two systems are believed to serve different physiological roles: the GDH is considered to be the low-affinity system, whereas the GS/GOGAT would be active as high-affinity system under nitrogen-limiting conditions.

In some organisms, GDH is the major system sustaining growth on ammonia, whereas the GS-GOGAT system is supposed to be active under nitrogen limitation only. Organisms from this group show a strongly reduced growth rate when the GDH activity is destroyed (Macheda et al., 1999). However, it has also been shown that this may be a matter of enzyme regulation, rather than intrinsic properties of the enzyme systems: for instance GS-GOGAT serves as the major uptake system under ammonia excess, provided that the culture pH is low (Buurman et al., 1989).

In contrast to this first group, GDH deficient mutants of S. *cerevisiae* show either a wild type phenotype or only a slightly reduced growth rate on ammonia depending on which of the two GDH activities that are present in S. *cerevisiae* was destroyed. Also GS/GOGAT deficient mutants have a wild type phenotype. Triple mutants, lacking both GDH activities and GS/GOGAT, are incapable of growth on ammonia and require glutamate for growth. This demonstrated that in this yeast, the presence of one of the two systems is sufficient for good growth on ammonia (Avendano et al., 1997).

In *Corynebacterium glutamicum* it has been shown that growth on ammonia and glutamate formation were not impaired in a GDH deficient mutant (Börmann-EI Kholy et al., 1993), although the maximal growth rate on ammonia was about 2 times lower (Tesch et al., 1999). Therefore, it is believed that the GS/GOGAT system is determining and limiting the maximal growth rate under these conditions. But even this lowered growth rate was sufficient to reach a cell density of 50 g/l within 35 hours which is sufficient for an efficient process design.

Other micro-organisms such as *Bacillus subtilis* and *Streptomyces clavuligerus* are lacking the GDH-system. *Bacillus subtilis* is able to grow fast on ammonia, although it lacks anabolic GDH activity. It also has a catabolic GDH but this functions in the opposite direction: it consumes glutamate as carbon-source and does not synthesise it (Belitsky and Sonenshein, 1998). It has been suggested that in *Streptomyces clavuligerus,* alanine dehydrogenase (ADH, EC 1.4.1.1), an enzyme that catalyses a similar reaction, may function instead of GDH in the assimilation of ammonia (Aharonowitz, 1979). The maximal growth rate of S. *clavuligerus* in a chemically defined medium is lower when ammonium salts are used as sole nitrogen source than when glutamate is used for this purpose. In fact, the growth rate on ammonium salts is extremely low. Whether or not this low growth rate is caused by the absence of GDH in this organism is not known.

The effect of lacking the GDH system differs from micro-organism to micro-organism and ranges from substantial effects to no effect at all. It seems that the regulatory strategy of the specific micro-organism is decisive here. It is quite unpredictable, therefore, what will happen if a GDH activity is introduced into an organism that normally does not possess this enzyme activity. The effect could be anything between stimulation of growth to actual inhibition of growth. This latter effect should especially be suspected if the GDH activity is introduced by a standard recombinant promoter which, in general, is not regulated by the nitrogen status of the cell. In addition, it could be suspected that this foreign enzyme activity is detrimental to product formation which in turn can be strongly related to the nitrogen status (Aharonowitz, 1979).

Surprisingly, we have found that transformation of micro-organisms that are lacking substantial GDH-activity with a GDH encoding gene, increases their growth rate when using ammonia as nitrogen source without exerting a negative effect on the formation of the secondary metabolites.

### References

Aharonowitz, Y. (1979) Regulatory interrelationships of nitrogen metabolism and cephalosporine biosynthesis. In: Genetics of industrial micro-organisms, pp. 210-217

Avendano, A., Deluna, A., Olivera, H., Valenzuela, L. and Gonzalez, A. (1997) GDH3 encodes a glutamate dehydrogenase isoenzyme, a previously unrecognised route for glutamate biosynthesis in *Saccharomyces cerevisiae*. *J.* Bacteriol. 179:5594-5597

Bailey, C.R. and Winstanley, D.J. (1986) Inhibition of restriction in *Streptomyces clavuligerus* by heat treatment. J. Gen. Microbiol. 132:2945-2947.

Belitsky, B.R. and Sonenshein, A.L. (1998) Role and regulation of *Bacillus subtilis* glutamate dehydrogenase genes. J. Bacteriol. 180:6298-6305

Bierman, M., Logan, R., O'Brien, K., Seno, E. T., Nagaraja, R. and Schoner, B.E. (1992) Plasmid cloning vectors for conjugal transfer of DNA from *Escherichia colt* to *Streptomyces* spp. Gene 116:43-49

Börmann-EI Kholy, E. R., Eikmanns, B.J. and Sahm, H. (1992) Molecular analysis of the *Corynebacterium glutamicum gdh* gene encoding glutamate dehydrogenase. Mol. Microbiol. 6:317-326

Börmann-EI Kholy, E. R., Eikmanns, B.J., Gutmann, M. and Sahm, H. (1993) Glutamate dehydrogenase is not essential for glutamate formation by *Corynebacterium glutamicum*. Appl. Environ. Microbiol. 59:2329-2331

Buurman, E. T., Teixeira de Mattos, M.J. and Neijssel, O. M. (1989) Nitrogen-limited behaviour of micro-organisms growing in the presence of large concentrations of ammonium ions. FEMS Microbiol. Lett. 58:229-232

Hopwood, D.A., Bibb, M.J., Chater, K.F., Kieser, T., Bruton, C.J., Kieser, H.M., Lydiate, D.J., Smith, C.P., Ward, J.M. and Schrempf, H. (1985) in: Genetic Manipulation *of Streptomyces:* a Laboratory Manual.

Illing, G.T., Normansell, I.D. and Peberdy, J.F. (1989) Protoplast isolation and regeneration in *Streptomyces clavuligerus.* J. Gen. Microbiol. 135: 2289-2297.

Leskiw, B.K., Aharonowitz, Y., Mevarech, M., Wolfe, S., Vining, L.C., Westlake, D.W.S. and Jensen, S. E. (1988) Cloning and nucleotide sequence determination of the isopenicillin N synthetase gene from *Streptomyces clavuligerus.* Gene 62:187-196

Macheda, M. L., Hynes, M.J. and Davis, M. A. (1999) The *Aspergillus nidulans gltA* gene encoding glutamate synthase is required for ammonium assimilation in the absence of NADP-glutamate dehydrogenase. Curr. Genet. 34:467-471

Motamedi, H., Shafiee, A. and Cai, S-J. (1995) Integrative vectors for heterologous gene expression in *Streptomyces* spp. Gene 160:25-31

Simon, R., O'Connell, M., Labes, M. and Pühler, A. (1986) Plasmid vectors for genetic analysis and manipulation of *Rhizobia* and other gram-negative bacteria. Methods Enzymol. 118:640-659

Stackebrandt, E., Rainey, F.A. and Ward-Rainey, N.L. (1997) Proposal for a new hierarchic classification system, *Actinobacteria* classis nov.. Int. J. Syst. Bacteriol. 47:479-491.

Tesch, M., Graaf, A.A. de, Sahm, H. (1999) *In vivo* fluxes in the ammonium-assimilatory pathways in *Corynebacterium glutamicum* studied by ¹⁵N Nuclear Magnetic Resonance. Appl. Environ. Microbiol. 65:1099-1109

### Description of the Figures

Figure 1. Physical map of the plasmid pSCG 1 that was used to transform S. *clavuligerus*.

Figure 2. Growth of transformant pSCG1-d and wild type (ATCC 27064) strains of S. *clavuligerus* in a defined medium containing ammonia as the sole nitrogen source (see Example 4). The cell density is measured as optical density at 550 nm.

### Summary of the invention

The present invention relates to a mutant micro-organism characterised by the fact that it possesses an improved capacity to assimilate ammonia compared to the corresponding wild type micro-organism.

In a second aspect, the invention relates to a DNA-construct comprising a DNA molecule encoding glutamate dehydrogenase that is obtainable from *Corynebacterium glutamicum*.

In another aspect, the invention relates to a process for the construction of mutant micro-organisms comprising the following steps:
- preparing a DNA-construct by cloning a, preferably heterologous, GDH-encoding DNA molecule, preferably obtainable from *Actinomycetales,* most preferably from *Corynebacterium glutamicum,* into a suitable vector;
- transforming a micro-organism with the DNA-construct thus obtained whereby the micro-organism is anyone of the group consisting of the groups of fungi, yeasts and bacteria, preferably belongs to the class of *Actinomycetales*, more preferably to the genus *Streptomyces,* most preferably *Streptomyces clavuligerus,;*
- selecting transformants on the basis of GDH-activity measured in cell free extracts obtained after culturing, harvesting and lysing cells by methods known in the art;
- selecting transformants on the basis of their growth rate in a medium containing ammonia as a nitrogen source and optionally by their capacity to produce secondary metabolites.

In a further aspect, the invention relates to a process for the production of secondary metabolites by culturing a mutant micro-organism that possesses an improved capacity to assimilate ammonia compared to the corresponding wild type micro-organism, characterised by the use of ammonia as a nitrogen source.

### Detailed description of the invention

The present invention solves the problem of how to culture micro-organisms in a fermentation medium containing ammonia as a nitrogen source while said micro-organisms are devoid of an efficient capacity to assimilate ammonia. It was surprisingly found that transformation of micro-organisms that are lacking substantial GDH-activity with a GDH-encoding gene, increases their growth rate when using ammonia as nitrogen source without exerting a negative effect on the formation of the secondary metabolites.

In one aspect, the invention relates to mutant micro-organisms characterised by an improved capacity to assimilate ammonia compared to the corresponding wild type micro-organism. The micro-organisms is anyone of the group consisting of the groups of fungi, yeasts and bacteria, preferably belongs to the class of *Actinomycetales* as defined by Stackebrandt *et al.* more preferably to the genus *Streptomyces.* Most preferred is *Streptomyces clavuligerus.*

In a second aspect, the invention relates to mutant micro-organisms that possess an improved capacity to assimilate ammonia compared to the corresponding wild type micro-organism and that are able to produce secondary metabolites, preferably clavulanic acid.

In a third aspect, the invention relates to mutant micro-organisms that possess an improved capacity to assimilate ammonia compared to the corresponding wild type micro-organism and possess and express a preferably heterologous DNA molecule encoding GDH while the corresponding wild type micro-organism is lacking substantial endogenous GDH activity. The DNA-molecule encoding said GDH is preferably obtainable from *Actinomycetales*, most preferably from *Corynebacterium glutamicum*.

With heterologous we mean that the organism from which the GDH-encoding DNA molecule is obtained, is not identical to the mutant micro-organism that, according to the present invention, possesses and expresses said GDH-encoding DNA molecule.

With a micro-organism that is lacking substantial endogenous GDH activity we mean that after transformation of said wild type micro-organism with a DNA-construct comprising a GDH-encoding DNA molecule, the GDH activity in the mutant micro-organism is improved with at least a factor 1.2, preferably at least a factor 2, more preferably at least a factor 5 and most preferably at least a factor 10 compared with the activity in the corresponding wild type micro-organism. This improvement factor depends on the absolute GDH activities in the wild type micro-organism as well as the mutant micro-organism, the latter being dependent on how the mutant micro-organism is transformed, e.g. on the number of GDH-gene copies. When the GDH-activity in the wild type micro-organism is very low or even undetectable, very high improvement factors can be obtained and vice versa. Usually, with more GDH-gene copies, higher GDH activities in the mutant micro-organism will be obtained.

In a fourth aspect, the invention provides a process for the construction of mutant micro-organisms possessing an improved capacity to assimilate ammonia compared to the corresponding wild type micro-organism, comprising the following steps:
- preparing a DNA-construct by cloning a, preferably heterologous, GDH-encoding DNA molecule, preferably obtainable from *Actinomycetales,* most preferably from *Corynebacterium glutamicum*, into a suitable vector;
- transforming a micro-organism with the DNA-construct thus obtained whereby the micro-organism is anyone of the group consisting of the groups of fungi, yeasts and bacteria, preferably belongs to the class of *Actinomycetales*, more preferably to the genus *Streptomyces*, most preferably *Streptomyces clavuligerus*,;
- selection of transformants on the basis of GDH-activity measured in cell free extracts obtained after culturing, harvesting and lysing cells by methods known in the art;
- selection of transformants on the basis of their growth rate in a medium containing ammonia as a nitrogen source and optionally by their capacity to produce secondary metabolites.

In a fifth aspect, the invention provides a DNA-construct comprising a GDH-encoding DNA molecule, preferably obtainable from *Actinomycetales*, most preferably from *Corynebacterium glutamicum*, that can be used to transform an endogenous GDH lacking wild type micro-organism that is anyone of the group consisting of the groups of fungi, yeasts and bacteria, preferably belongs to the class of *Actinomycetales*, more preferably to the genus *Streptomyces,* most preferably *Streptomyces clavuligerus.*

In a sixth aspect of the invention, a process is provided for the production of secondary metabolites, preferably clavulanic acid, comprising culturing a mutant micro-organism that possesses an improved capacity to assimilate ammonia compared to the corresponding wild type micro-organism, characterised by the use of ammonia as a nitrogen source, preferably the sole nitrogen source.

### Example 1

### Cloning of the gene encoding Glutamate Dehydrogenase (GDH) from Corynebacterium glutamicum into a Streptomyces/E. coli shuttle vector.

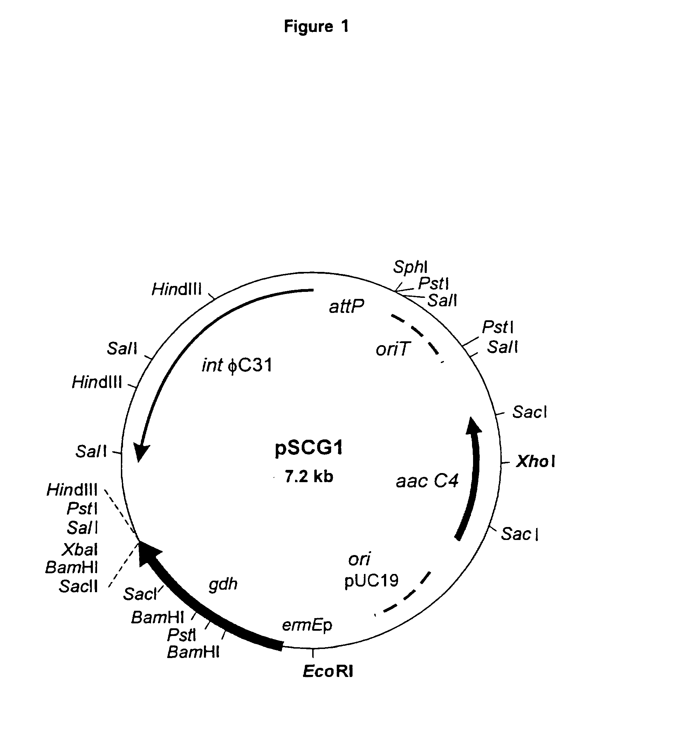

The *gdh* gene from *C*. *glutamicum* (Börmann *et al*. 1992) was amplified using Pfu polymerase (StrataGene) and the DNA oligonucleotides GDH3 (SEQ01: 5' GTC*GAATTCATATG*ACAGTTGATGA 3', nt 1 to 19 of *gdh, Eco*RI and *Nde*l sites are italicised) and GDH5 (SEQO2: 5'GTC*AAGCTT*GTGGATCGGATAATTGACC 3', nt 84 to 64 downstream of stopcodon of *gdh, Hin*dlll site is italicised). Chromosomal DNA was isolated from *C. glutamicum* according to Hopwood *et al.* 1985) and used as a template in the PCR reaction (35 cycles 30s at 94°C, 90s at 54°C, 120s at 72°C). The PCR product was digested with *Eco*RI and *Hin*dIII and cloned as a 1.5 kb fragment into the *Eco*RI/*Hin*dIII sites of pUC18: **pUCG4**. Subsequently, a 1.5 kb *Nde*l/*Hin*dIII fragment of pUCG4 was cloned into the *Nde*I/*Hin*dIII sites of pHM10A (Motamedi *et al*. 1995): **pHCG2**. A 1.7 kb *Eco*RI/*Hin*dIII fragment of pHCG2, with the *Hin*dIII site filled in using Klenow fragment of DNA polymerase I was cloned into the *Eco*RI/*Eco*RV sites of pSET52 (Bierman *et al*. 1992), to yield **SCG1** (see the physical map in Figure 1).

### Example 2

### Transformation of Streptomyces clavuligerus with pSCG1.

Protoplasts of *S*. *clavuligerus* ATCC 27064 or a strain derived thereof by mutation and selection for higher clavulanic acid productivity were isolated as described by Illing *et al.* (1989). The protoplasts were washed and resuspended in medium P having a composition as used in the R5 protocol (Illing, *supra*), with an additional 1 % BSA. Prior to transformation, the protoplasts were heat-treated at 43°C for 10 minutes (Bailey *et al*. 1986). Transformations were carried out using ca. 10⁹ protoplasts and 25% (w/v) polyethylene glycol (PEG 1000) in medium P without BSA, following the procedure as described by Hopwood *et al*. (1985, pp. 110-111). Before plating on regeneration medium, the protoplasts were washed in medium P and resuspended in the same containing 1 % BSA. Transformants were selected by overlaying the plates with 2.5 ml soft nutrient agar containing 400 µg/ml apramycin.

The regeneration medium (Leskiw *et al*. (1988) - with minor modifications) contained (in gram per litre tap water): sucrose, 160; K₂SO₄, 0.1; MgSO₄.7 H₂O, 0.05; casamino acids, 1; yeast extract, 5; maltose, 10; 1 ml trace elements cocktail (see below) and 250 ml 0.1 M 3-(N-morpholino)-propanesulfonic acid (MOPS) buffer pH 7.2. After sterilisation, 100 ml of a 10% sodium glutamate solution and 13.5 ml of a 5 M CaCl₂ solution were added per litre. The trace element cocktail (Hopwood *et al*., *supra)* contained in mg per litre: ZnCl₂, 40; FeCl₃.6H₂O, 200; CuCl₂.2H₂O, 10; MnCl₂.4H₂O, 10; Na₂B₄O₇.1OH₂O, 10; (NH₄)₆Mo₇O₂₄.4H₂O, 10.

In an alternative procedure, the *gdh* gene containing shuttle plasmid was introduced into S. *clavuligerus* by conjugation with *E. coil* donor strain S17-1 (Simon *et al*.) containing the same. The latter strain was grown till an OD₅₆₀ of 0.5 and 300 µl of the culture was mixed with 10⁸ pre-germinated S. *clavuligerus* spores contained in the same volume of 2xYT medium containing in gram per litre tap water: tryptone, 16; yeast extract, 10; NaCI, 5. Pre-germination of the spores was performed by incubation at 50°C for 10 minutes in 2xYT medium, followed by a further incubation at 28°C for at least 30 minutes. The conjugation mixture was centrifuged, the pellet resuspended in 2xYT medium and plated on SFM medium containing in gram per litre: soy flour, 20; maltose, 20; bacto-agar, 20 with 10 mM MgCl₂. Transformants were selected by overlaying the plates with 1 ml H₂O containing 0.25 mg nalidixic acid and 0.5 mg apramycin.

### Example 3

### Expression of GDH in transformed S. clavuligerus

Transformants selected in Example 2 were grown in 20 ml cultures TSB medium (30 g/l Tryptone Soya Broth - Oxoid) plus 1% maltose for 30-40 hours at 28°C. The mycelia were harvested, washed with 0.2 M potassium phosphate pH 7.0 and resuspended in 2-3 ml of the same buffer. Cell-free extracts were prepared by sonication and subsequent centrifugation at 13,000 rpm for 30 minutes at 4°C. GDH activity in the extracts was measured by following spectrophotometrically the decrease in absorbance at 340 nm in a reaction volume of 1 ml containing 100 mM Tris/HCI pH 8.0, 0.25 mM NADPH, 20 mM NH₄Cl, 10 mM alpha-ketoglutarate (Börmann et al., *supra*). Cell-free extract was added until a linear decrease in absorbance was obtained. Units of GDH activity were calculated from the absorption coefficient at 340 nm of NADPH (6.22 mM⁻¹.cm⁻¹). One GDH unit is defined as the amount of enzyme catalysing the conversion of 1 nmole of substrate per minute at ambient temperature. Typical activities obtained with positive transformants (in units/mg total protein) are given in the Table 1.

**Table 1**

| Strain Strain | GDH activity units/mg total protein |
|---|---|
| Control (ATCC 27064) | not detectable |
| Transformants | |
| SCG1-a | 1267 |
| SCG1-b | 1241 |
| SCG1-c | 2712 |
| SCG1-d | 1541 |
| SCG1-e | 1285 |

### Example 4

### Growth of S. clavuligerus expressing GDH on defined medium with ammonia as the nitrogen source.

Spores of S. *clavuligerus* (wild type and transformant SCG1-d) were inoculated in 20 ml TSB (*supra*) plus 1% maltose and incubated for 18-20 hours at 28°C at 300 rpm. Prior to inoculation of this preculture to defined medium, the mycelia were washed three times with minimal medium without nitrogen source. Washed mycelia were inoculated into 20 ml minimal medium in 100 ml Erlenmeyer flasks and growth at 28°C was followed for several days.

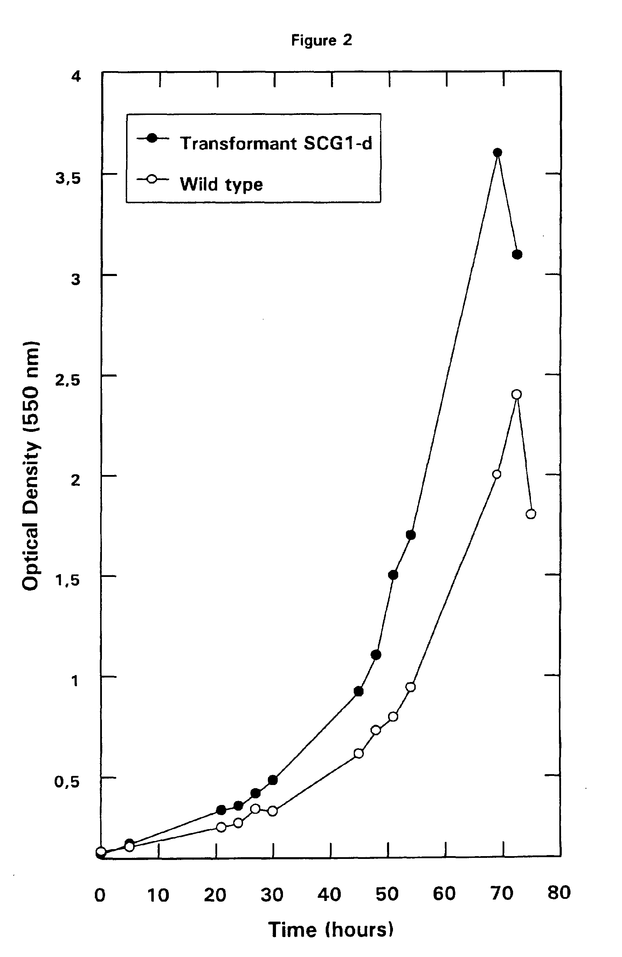

The medium contained in gram per litre tap water: glycerol, 30; KH₂PO₄, 4.5; MgSO₄.7H₂O, 0.5; MOPS, 20; (NH4)2S04, 2 and 2 ml per litre of the trace element cocktail containing 1 gram per litre each of ZnCl₂, FeSO₄.7H₂O, MnCl₂.4H₂O, CaCl₂.2H₂O. The growth curves are shown in Figure 2.

### Example 5

### Clavulanic acid production by GDH-expressing transformants

*Streptomyces clavuligerus* ATCC 27064 and GDH expressing transformants were inoculated in 100 ml shake flasks containing 20 ml of a preculture medium containing in gram per litre: (NH₄)₂SO₄, 2.75; KH₂PO₄, 0.5; MgSO₄.7H₂O, 0.5; CaCl₂.2H₂O, 0.01; MOPS, 21; glycerol, 19.9; sodium succinate, 5.5 and 0.06 ml per litre of a trace elements solution containing in gram per litre: ZnSO₄.7H₂O, 2.8; ferric ammonium citrate, 2.7; CuSO₄.5H₂O, 0.125; MnSO₄.2H₂O, 1; CoCl₂.6H₂O, 0.1; Na₂B₄O₇.10H₂O, 0.16; Na₂MoO₄.2H₂O, O.054). The cultures were incubated in an orbital shaker at 220 rpm at 28°C for 48 hours.

1 ml of the cultures was used to inoculate in 100 ml shake flasks 20 ml of a production medium containing in gram per litre: (NH₄)₂SO₄, 2; asparagine, 4; KH₂PO₄, 1.5; MgSO₄.7H₂O, 0.5; CaCl₂.2H₂O, 0.01; MOPS 21; glycerol, 19.9; sodium succinate, 5.5 and 0.06 ml per litre of a trace elements solution containing in gram per litre: FeSO₄.7H₂O, 0.5; MnCl₂.4H₂O, 0.1; ZnSO₄.7H₂O, 0.1. The incubation was continued for 96 hours. At the end of the fermentation, the mycelium was removed by centrifugation and the clavulanic acid concentration in the supernatant fraction was determined by HPLC methods well known in the art.

The clavulanic acid production of 11 independent transformants were compared with the production level of S. *clavuligerus* ATCC 27064. Table 2 shows the results (the production level of strain ATCC 27064 is set to 100%):

**Table 2.**

| strain | clavulanic acid production level (%) |
|---|---|
| Control (ATCC 27064) | 100 |
| Transformant | |
| SCG1-1 | 106 |
| SCG1-2 | 73 |
| SCG1-3 | 99 |
| SCG1-4 | 102 |
| SCG1-5 | 107 |
| SCG1-6 | 101 |
| SCG1-7 | 74 |
| SCG1-8 | 69 |
| SCG1-9 | 104 |
| SCG1-10 | 108 |
| SCG1-11 | 108 |

The variation in production level for different independent transformants is comparable to the variation found when different untransformed isolates of ATCC 27064 (not shown). Table 2 shows that the transformants are not impaired in their clavulanic acid production capacity.

## Claims

1. A mutant micro-organism characterised by an improved capacity to assimilate ammonia compared to the corresponding wild type micro-organism.

2. The micro-organism according claim 1 wherein the mutant micro-organism possesses and expresses a preferably heterologous DNA molecule encoding glutamate dehydrogenase while the corresponding wild type micro-organism is lacking substantial endogenous glutamate dehydrogenase activity.

3. The micro-organism according to claims 2 wherein the DNA molecule encoding glutamate dehydrogenase is obtainable from *Actinomycetales.*

4. The micro-organism according to any one of the claims 2 or 3 wherein the DNA molecule encoding glutamate dehydrogenase is obtainable from *Corynebacterium glutamicum*.

5. The micro-organism according to any one of the claims 1-4 wherein the micro-organism is anyone of the group consisting of the groups of fungi, yeasts and bacteria.

6. The micro-organism according to any one of the claims 1-5 wherein the micro-organism belongs to the class of *Actinomycetales.*

7. The micro-organism according to any one of the preceding claims wherein the micro-organism belongs to the genus *Streptomycetes.*

8. The micro-organism according to any one of the preceding claims wherein the micro-organism is *Streptomyces clavuligerus.*

9. The micro-organism according to any one of the preceding claims wherein the micro-organism is able to produce secondary metabolites.

10. The micro-organism according to claim 9 wherein the secondary metabolite is clavulanic acid.

11. A DNA-construct comprising a DNA molecule encoding glutamate dehydrogenase that is obtainable from *Corynebacterium glutamicum.*

12. A process for the construction of mutant micro-organisms as defined in claims 2-10 comprising the following steps
- preparing a DNA-construct as defined in claim 11 by cloning a, preferably heterologous, GDH-encoding DNA molecule, preferably obtainable from *Actinomycetales*, most preferably from *Corynebacterium glutamicum,* into a suitable vector;
- transforming a micro-organism with the DNA-construct thus obtained whereby the micro-organism is anyone of the group consisting of the groups of fungi, yeasts and bacteria, preferably belongs to the class of *Actinomycetales,* more preferably to the genus *Streptomyces,* most preferably *Streptomyces clavuligerus*;
- selecting transformants on the basis of GDH-activity measured in cell free extracts obtained after culturing, harvesting and lysing cells by methods known in the art;
- selecting transformants on the basis of their growth rate in a medium containing ammonia as a nitrogen source and optionally by their capacity to produce secondary metabolites.

13. A process for the production of secondary metabolites comprising culturing a micro-organism defined in claims 9-10 characterised by the use of ammonia as a nitrogen source for the production of the metabolite.

14. A process for the production of secondary metabolites according to claim 13 characterised by the use of ammonia as the sole nitrogen source.

15. A process according to claims 13-14 characterised by the fact that the secondary metabolite is clavulanic acid.
